# EUROPEAN PATENT APPLICATION

(11) **EP 4 516 769 A1**
(43) Date of publication of application: **05.03.2025**
(21) Application number: 23796042.2
(22) Date of filing: 06.04.2023
(51) Int. Cl.: C07C 29/36, B01J 31/22, C07C 29/80, C07C 31/10, C07B 61/00

(54) **METHOD FOR PRODUCING ALCOHOL**

(30) Priority: 27.04.2022 JP 2022073646
(71) Applicant: Resonac Corporation, Tokyo 105-7325 (JP)
(72) Inventor: FUJIBE, Satoshi, Tokyo 105-8518 (JP); INOUE, Gen, Tokyo 105-8518 (JP); TAKAHASHI, Yuta, Tokyo 105-8518 (JP)
(74) Representative: Strehl Schübel-Hopf & Partner
(86) International application number: PCT/JP2023/014269
(87) International publication number: WO 2023/210296

(57) **Abstract**

Provided is a method that is for producing an alcohol from an olefin compound with an improved selectivity and that enables a catalyst to be easily reused. This method for producing an alcohol comprises: reacting an olefin compound, carbon monoxide, and hydrogen molecules, using a group-9 transition metal complex as a catalyst, in an organic solvent containing at least 30 mol% of an amine compound having at least two nitrogen atoms that form a tertiary amine.

## Description

### TECHNICAL FIELD

The present invention relates to a method for producing an alcohol from an olefin compound in one-step process.

### BACKGROUND

The hydroformylation reaction of an olefin compound with carbon monoxide and a hydrogen molecule to produce an aldehyde has high industrial utility value. In particular, propionaldehyde and butyraldehyde obtained from ethylene and propylene, respectively, are further converted to alcohols, such as 1-propanol and butanol, and such an alcohol is widely used as a raw material for various products, such as an industrial solvent, and a medical and agricultural chemical.

In the hydroformylation reaction, generally, a cobalt carbonyl complex catalyst, a cobalt phosphine complex catalyst, a rhodium carbonyl complex catalyst, a rhodium phosphine complex catalyst, etc., are used, and the reaction is carried out in a state in which the complex catalyst is homogeneously dissolved in a solvent. Both cobalt and rhodium are expensive metals with little production, and are required to be recovered and repeatedly used from the viewpoint of manufacturing cost.

The main product in the hydroformylation reaction of an olefin compound is an aldehyde in which one-carbon unit is added to the olefin compound, and it is necessary to reduce the aldehyde in order to produce a useful alcohol. Usually, the reduction is carried out using a different type of catalyst from that used for the hydroformylation reaction in a separate reactor from the hydroformylation reaction, so that two catalysts and two reaction processes are required to produce an alcohol from an olefin compound. Further, after the hydroformylation process, complicated steps including recovery of the hydroformylation catalyst, and removal of carbon monoxide to prevent deactivation of the catalyst used in the reduction process are required. Therefore, there is a need for a technique for producing an alcohol from an olefin compound using one catalyst in one-step process.

In this connection, it is known that an alcohol is produced from an olefin compound in one-step process by adding trialkylphosphine to a cobalt complex catalyst (Patent Literature 1; US 3420898 A). However, in this method, since a large amount of phosphine compound is used, there is a concern about toxicity, and in addition, a high temperature exceeding 180°C is required in order to increase the alcohol selectivity. Therefore, when an olefin compound having a small number of carbon atoms, such as ethylene and propylene, is used as a raw material, there is a problem that the pressure becomes extremely high under high temperature conditions, which makes the production difficult.

A rhodium complex catalyst is known as a catalyst that can react under milder conditions. For example, a gas phase reaction is known in which ethylene, carbon monoxide, and hydrogen are brought into contact with a catalyst in which a cobalt compound and a rhodium compound are supported on alumina to produce 1-propanol (Patent Literature 2; JP H08-790 B). However, there is a problem that the 1-propanol selectivity is low, since the preparation of ethane by hydrogenation of ethylene proceeds simultaneously. As a reaction in a liquid phase, a reaction for obtaining 1-propanol using a rhodium triethylphosphine complex catalyst is also known (Non-Patent Literature 1; J. Chem. Soc., Dalton Trans. 1996, 1161). However, since a phosphine ligand is used, there is a concern about toxicity.

For a rhodium complex catalyst without a phosphine ligand, a system is known in which hexene or octene is reacted in a mixed solvent of water and amine using Rh₂O3 as a catalyst (Non-Patent Literature 2; Chem. Ing. Tech. 1972, 44(11), 708). However, there is a problem in that the purity of the obtained alcohol cannot be increased due to water used as the solvent.

For a reaction in an organic solvent, a reaction of octene in a system in which a tertiary amine is added to Rh(acac)(CO)₂ is known (Non-Patent Literature 3; J. Catal. 2021, 400, 234). However, the Rh catalyst, which is expensive, is required in large quantities for industrial alcohol production, since the turnover per unit time, (Turnover Frequency, TOF) of the Rh catalyst for alcohol production is as low as 93 h⁻¹. Although a system in which N,N,N',N'-tetramethylethylenediamine or N,N,N',N'-tetramethylbutanediamine having two nitrogen atoms constituting a tertiary amine in the molecular is added is also studied, the alcohol yield is less than 10%. In Non-Patent Literature 3, a reaction system using a solvent amount of triethylamine is also studied. However, the alcohol yield is reported to be only 23%. Therefore, development of a catalyst system having high activity under mild conditions has been required.

### [Citation List]

### [Patent Literature]

[PTL 1] US 3420898 A
[PTL 2] JP H08-790 B

### [Non-Patent Literature]

[NPL 1] J. Chem. Soc., Dalton Trans. 1996, 1161
[NPL 2] Chem. Ing. Tech. 1972, 44(11), 708
[NPL 3] J. Catal. 2021, 400, 234

### SUMMARY OF INVENTION

### [Technical Problem]

It is an object of the present invention to provide a method for producing an alcohol from an olefin compound in which the alcohol can be produced with improved selectivity, and a catalyst can be easily reused.

### [Solution to Problem]

As a result of intensive studies to solve the aforementioned problems, the present inventors have found that an alcohol can be produced with improved selectivity by reacting an olefin compound, carbon monoxide, and a hydrogen molecule in an organic solvent containing a transition metal complex catalyst of Group 9 and a specific amine compound at a concentration more than or equal to a certain concentration, and the catalyst can be easily reused, thereby completing the present invention.

That is, the present invention relates to the following [1] to [13].
[1] A method for producing an alcohol comprising reacting an olefin compound, carbon monoxide, and a hydrogen molecule in an organic solvent containing 30 mol% or more of an amine compound having two or more nitrogen atoms constituting a tertiary amine, using a transition metal complex of Group 9 as a catalyst.
[2] The method for producing an alcohol according to [1], wherein the amine compound having two or more nitrogen atoms constituting a tertiary amine is represented by formula (1) or formula (2): 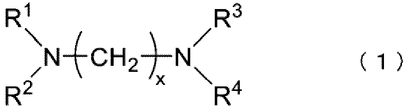 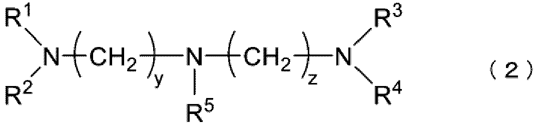 wherein, R¹ to R⁵ each independently represent an alkyl group having 1 to 10 carbon atoms, and x, y, and z each independently represent an integer of 2 to 10.
[3] The method for producing an alcohol according to [2], wherein in formula (1) or (2), R¹ to R⁵ are all methyl groups or all ethyl groups, and x, y, and z are each independently an integer of 2 to 6.
[4] The method for producing an alcohol according to [1], wherein the amine compound having two or more nitrogen atoms constituting a tertiary amine is at least one selected from the group consisting of N,N,N',N'-tetramethylethylenediamine, N,N,N',N'-tetramethyl-1,3-propanediamine, N,N,N',N'-tetramethyl-1,4-butanediamine, N,N,N',N'-tetramethyl-1,6-hexanediamine, and N,N,N',N",N"-pentamethyldiethylenetriamine.
[5] The method for producing an alcohol according to [1], wherein the amine compound having two or more nitrogen atoms constituting a tertiary amine is N,N,N',N'-tetramethyl-1,3-propanediamine.
[6] The method for producing an alcohol according to any one of [1] to [5], wherein the transition metal complex of Group 9 is a metal complex composed of acetylacetonatodicarbonyl and a transition metal of Group 9.
[7] The method for producing an alcohol according to any one of [1] to [6], wherein the transition metal of Group 9 is rhodium.
[8] The method for producing an alcohol according to any one of [1] to [7], wherein the organic solvent containing 30 mol% or more of the amine compound having two or more nitrogen atoms constituting a tertiary amine is an organic solvent comprising the amine compound having two or more nitrogen atoms constituting a tertiary amine, and at least one selected from the group consisting of toluene, xylene, and n-butanol.
[9] The method for producing an alcohol according to any one of [1] to [8], wherein the molar ratio of the amine compound having two or more nitrogen atoms constituting a tertiary amine to the transition metal of Group 9 is 500 to 10,000.
[10] The method for producing an alcohol according to any one of [1] to [9], wherein the olefin compound is a monoolefin having 2 to 4 carbon atoms.
[11] The method for producing an alcohol according to any one of [1] to [10], wherein the olefin compound is ethylene and the product is 1-propanol.
[12] The method for producing an alcohol according to any one of [1] to [11], comprising separating a produced alcohol after the reaction, recovering the organic solvent containing the transition metal complex of Group 9, adding an olefin compound, carbon monoxide, and a hydrogen molecule to the recovered organic solvent, and reacting the mixture.
[13] The method for producing an alcohol according to [12], comprising distilling off the produced alcohol by distillation at a temperature of less than 200°C, and recovering the organic solvent containing a transition metal complex of Group 9.

### [Advantageous Effects of Invention]

According to the present invention, it is possible to provide a method for producing an alcohol from an olefin compound in which the alcohol can be produced with improved selectivity, and a used catalyst can be easily reused.

### DESCRIPTION OF EMBODIMENTS

In the present specification, when using "to" for a numerical range, the numerical values at both ends are the upper limit value and the lower limit value, respectively, and are included in the numerical range.

A method for producing an alcohol of one embodiment is a method in which the alcohol can be produced in a highly selective manner, and a used catalyst can be easily reused by reacting an olefin compound, carbon monoxide, and a hydrogen molecule in an organic solvent containing 30 mol% or more of an amine compound having two or more nitrogen atoms constituting a tertiary amine in the presence of a transition metal complex of Group 9.

### [Alcohol production reaction]

The reaction of an olefin compound, carbon monoxide, and a hydrogen molecule produces an alcohol having one more carbon atom than the olefin compound of the raw material. A transition metal complex of Group 9 is used as the catalyst. As the solvent, an organic solvent containing 30 mol% or more of an amine compound having two or more nitrogen atoms constituting a tertiary amine is used.

The reaction formula in the case where the olefin compound is ethylene and the produced alcohol is 1-propanol is shown below.

CH₂=CH₂ + CO + 2H₂ → CH₃-CH₂-CH₂OH

The reaction proceeds in two steps as follows.
(first step)

   CH₂=CH₂ + CO + H₂ → CH₃-CH₂-CHO
(second step)

   CH₃-CH₂-CHO + H₂ → CH₃-CH₂-CH₂OH

In general, the transition metal complex of Group 9 acts as a catalyst for the first step reaction but not for the second step reaction. However, without wishing to be bound by theory, in the method of the present invention, by the presence of an amine compound having two or more nitrogen atoms constituting a tertiary amine at a certain concentration or more, the mechanism of action is unknown, but the transition metal complex of Group 9 is activated, and the transition metal complex of Group 9 acts as a catalyst in both the first step and the second step. Thus, an alcohol can be produced using one catalyst in a highly selective manner from an olefin compound in one-step process. When the number of carbon atoms of the olefin compound is 3 or more, isomers for the produced alcohol may generate depending on the position at which carbon monoxide is added.

### [Olefin compound]

An olefin compound of one embodiment is a monoolefin having one carbon-carbon double bond. The olefin compound is preferably a monoolefin having 2 to 20 carbon atoms, and more preferably a monoolefin having 2 to 4 carbon atoms. Specific examples thereof include acyclic aliphatic olefin compounds, such as ethylene, propylene, butene, pentene, and hexene, alicyclic olefin compounds, such as cyclobutene, cyclopentene, and cyclohexene, and aromatic olefin compounds, such as styrene, and indene. An olefin compound of one embodiment is preferably an α-olefin from the viewpoint of increasing the yield. Ethylene, propylene, and butene are preferable from the viewpoint of producing an industrially useful alcohol. When the number of carbon atoms of the olefin compound is 3 or more, the produced alcohol has isomers. Ethylene is particularly preferable, since the isomeric alcohol is not produced.

### [Transition metal complex of Group 9]

In a transition metal complex of Group 9 of one embodiment, the transition metal of Group 9 is cobalt (Co), rhodium (Rh), iridium (Ir), or meitnerium (Mt), preferably cobalt (Co), rhodium (Rh), or iridium (Ir). From the viewpoint of improving the alcohol selectivity, rhodium (Rh) is particularly preferable.

Examples of the ligand constituting the transition metal complex of Group 9 include acetylacetonato, halogen, carbonyl, cyclooctadienyl, triphenylphosphine, and hydrido, and acetylacetonato, halogen, carbonyl, and cyclooctadienyl are preferable, and, from the viewpoint of improving the stability of the complex, acetylacetonato and carbonyl are more preferable. From the viewpoint of suppressing toxicity, it is preferable not to use phosphine.

Specific examples of the transition metal complex of Group 9 include acetylacetonatodicarbonylrhodium (Rh(acac)(CO)₂), hexadecacarbonylhexarhodium (Rh₆(CO)₁₆), cyclooctadiene rhodium chloride dimer ([RhCl(cod)]₂), 'acetylacetonatocarbonyltriphenylphosphine rhodium (Rh(acac)(CO)(PPh₃)), hydridocarbonyltris(triphenylphosphine)rhodium (RhH(CO)(PPh₃)₃), acetylacetonatodicarbonyliridium (Ir(acac)(CO)₂), hydridocarbonyltris(triphenylphosphine)iridium (IrH(CO)(PPh₃)₃), etc. From the viewpoint of improving the alcohol selectivity and suppressing toxicity, the transition metal complex of Group 9 is preferably a metal complex composed of acetylacetonatodicarbonyl and a transition metal of Group 9, and more preferably acetylacetonatodicarbonylrhodium. The transition metal complex of Group 9 may be used alone, or in combination of two or more thereof.

### [Amine compound having two or more nitrogen atoms constituting a tertiary amine]

An amine compound having two or more nitrogen atoms constituting a tertiary amine of one embodiment (hereinafter sometimes referred to as "amine compound") is not particularly limited as long as it is a compound having two or more nitrogen atoms constituting a tertiary amine. The number of nitrogen atoms constituting a tertiary amine present in the compound is preferably 2 to 4, and more preferably 2 or 3. The nitrogen atom constituting a tertiary amine is a nitrogen atom covalently bonded to three hydrocarbon groups. The hydrocarbon group may be an aliphatic group, an aromatic group, or a group composed of a combination thereof. The nitrogen atoms constituting a tertiary amine may constitute a dialkylamino group (-NRR'; R, and R' are alkyl groups.) or an alkylimino group (-NR"-; R" is an alkyl group.). The two alkyl groups of the dialkylamino group may be identical or different, but are preferably identical for ease of availability. When two or more dialkylamino groups are present in the amine compound, the dialkylamino groups may be identical or different, but are preferably identical for ease of availability. The number of carbon atoms of the alkyl group in the dialkylamino group and the alkylimino group is preferably 1 to 10. Specifically, a methyl group and an ethyl group are preferable from the viewpoint of improving the alcohol yield. The amine compound having two or more nitrogen atoms constituting a tertiary amine may be used alone, or in combination of two or more thereof.

The amine compound having two or more nitrogen atoms constituting a tertiary amine is preferably a compound represented by formula (1) or the formula (2): 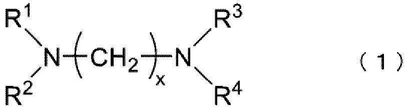 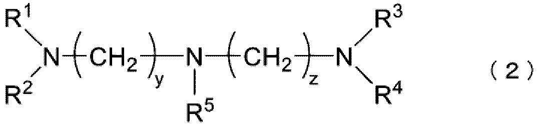 wherein, R¹ to R⁵ each independently represent an alkyl group having 1 to 10 carbon atoms, and x, y, and z each independently represent an integer of 2 to 10.

It is preferable that R¹ to R⁵ each independently be an alkyl group having 1 to 6 carbon atoms, more preferably a methyl group or an ethyl group, and particularly preferably a methyl group. It is preferable that R¹ to R⁵ be all methyl groups or all ethyl groups. x is an integer of 2 to 10, preferably an integer of 2 to 6, and more preferably 3. y, and z are each independently an integer of 2 to 10, preferably an integer of 2 to 6, and more preferably 2. Particularly preferred amine compounds are those in formula (1) or (2) in which R¹ to R⁵ is all methyl groups or all ethyl groups, and x, y and z are each independently an integer of 2 to 6.

The amine compound having two or more nitrogen atoms constituting a tertiary amine is preferably liquid in the range of 20 to 150°C and 1 to 200 atm from the viewpoint of ease of separating from the produced alcohol.

Specific examples of the amine compound having two or more nitrogen atoms constituting a tertiary amine include those having two nitrogen atoms, such as N,N,N',N'-tetramethylethylenediamine, N,N,N',N'-tetraethylethylenediamine, N,N,N',N'-tetramethyl-1,3-propanediamine, N,N,N',N'-tetramethyl-1,4-butanediamine, N,N'-diethyl-N,N'-dimethyl-1,4-butanediamine, N,N,N',N'-tetramethyl-1,6-hexanediamine, N,N,N',N'-tetramethyl-1,2-propanediamine, N,N,N',N'-tetramethyl-2-methyl-1,3-propanediamine, N,N,N',N'-tetraethyl-2,2-dimethyl-1,3-propanediamine, and N,N'-diethyl-N,N'-dimethyl-3-methyl-2,4-butanediamine.

Those having three nitrogen atoms include N,N,N',N",N"-pentamethyldiethylenetriamine, N,N"-diethyl-N,N',N"-trimethyldiethylenetriamine, etc.

Among these, N,N,N',N'-tetramethylethylenediamine, N,N,N',N'-tetramethyl-1,3-propanediamine, N,N,N',N'-tetramethyl-1,4-butanediamine, N,N,N',N'-tetramethyl-1,6-hexanediamine, and N,N,N',N",N"-pentamethyldiethylenetriamine are preferable, and N,N,N',N'-tetramethyl-1,3-propanediamine, N,N,N',N'-tetramethyl-1,4-butanediamine, N,N,N',N'-tetramethyl-1,6-hexanediamine, and N,N,N',N",N"-pentamethyldiethylenetriamine are more preferable, and N,N,N',N'-tetramethyl-1,3-propanediamine is particularly preferable, from the viewpoint of ease of availability.

### [Method for producing alcohol]

In a method for producing an alcohol of one embodiment, an organic solvent containing 30 to 100 mol% of an amine compound having two or more nitrogen atoms constituting a tertiary amine is used. The organic solvent is preferably composed only of the amine compound having two or more nitrogen atoms constituting a tertiary amine. The organic solvent may be a mixed solvent of the amine compound and other organic solvent.

The other organic solvent is preferably an organic solvent that does not affect the reaction. Specific examples thereof include acyclic aliphatic hydrocarbons, such as hexane, octane, decane, and dodecane; alicyclic hydrocarbons, such as cyclohexane, methylcyclohexane, and cyclododecane; aromatic hydrocarbons, such as benzene, toluene, xylene, and mesitylene; halogenated hydrocarbons, such as chloroform, and 1,2-dichloroethane; esters, such as ethyl acetate, and n-propyl acetate; ethers, such as diethyl ether, and anisole; ketones, such as methyl ethyl ketone, and cyclohexanone; alcohols, such as ethanol, n-propanol, n-butanol, and isobutanol; and aldehydes, such as propionaldehyde, n-butyraldehyde, and isobutyraldehyde. Among these, acyclic aliphatic hydrocarbons, aromatic hydrocarbons, and alcohols are preferable, decane, toluene, xylene, and n-butanol are more preferable, and toluene, xylene, and n-butanol are particularly preferable. An organic solvent comprising the amine compound having two or more nitrogen atoms constituting a tertiary amine and at least one selected from the group consisting of toluene, xylene, and n-butanol is also preferably used. It is also preferable to mix an alcohol and an aldehyde obtained from an olefin compound, carbon monoxide and a hydrogen molecule used in the reaction. The other organic solvent may be used alone, or in combination of two or more thereof. The organic solvent used in the method for producing an alcohol preferably does not contain an inorganic solvent, such as water from the viewpoint of increasing the purity of the obtained alcohol. The content of the inorganic solvent in the organic solvent may be 1 mol% or less, 0.5 mol% or less, or 0.1 mol% or less.

In a method for producing an alcohol of one embodiment, the content of the amine compound having two or more nitrogen atoms constituting a tertiary amine in the organic solvent (amine compound (mol) / organic solvent (mol) × 100) is 30 to 100 mol%, preferably 60 to 100 mol%, and more preferably 80 to 100 mol%. When the content is less than 30 mol%, the alcohol selectivity may decrease.

In a method for producing an alcohol of one embodiment, the molar ratio of the amine compound having two or more nitrogen atoms constituting a tertiary amine to the transition metal of Group 9 (amine compound (mol) / transition metal of Group 9 (mol)) is preferably 500 to 10,000, more preferably 500 to 5,000, and particularly preferably 500 to 2,000. When it is 500 to 10,000, the alcohol selectivity does not decrease.

In a method for producing an alcohol of one embodiment, the molar ratio of carbon monoxide to the olefin compound (carbon monoxide (mol) / olefin compound (mol)) is, for example, 1 to 5, preferably 1.2 to 4, and more preferably 1.5 to 3. When it is 1 or more, the total yield of aldehyde and alcohol increases, and when it is 5 or less, the alcohol selectivity increases.

In a method for producing an alcohol of one embodiment, the molar ratio of the hydrogen molecule to the olefin compound (hydrogen molecule (mol) /olefin compound (mol)) is, for example, 2 to 10, preferably 2 to 5, and more preferably 2 to 3. When it is 2 or more, the total yield of aldehyde and alcohol increases, and when it is 10 or less, the alcohol selectivity increases.

In a method for producing an alcohol of one embodiment, the molar ratio of the olefin compound to the transition metal complex of Group 9 (olefin compound (mol) / transition metal complex of Group 9 (mol)) is, for example, 100 to 100,000, preferably 200 to 20,000, and more preferably 500 to 10,000. When it is 100 or more, the production cost of alcohol is suppressed, and when it is 100,000 or less, the alcohol yield can be increased.

In a method for producing an alcohol of one embodiment, the reaction temperature is, for example, 20°C to 200°C, preferably 50°C to 130°C, and more preferably 70°C to 110°C. When the reaction temperature is 20°C or more, the total yield of aldehyde and alcohol increases, and when the reaction temperature is 200°C or less, the alcohol selectivity increases.

In a method for producing an alcohol of one embodiment, the reaction pressure is, for example, 20 to 200 atm, preferably 50 to 150 atm, and more preferably 70 to 130 atm. When the reaction pressure is 20 atm or more, the alcohol yield increases, and when the reaction pressure is 200 atm or less, the alcohol selectivity increases. When carbon monoxide and a hydrogen molecule are replenished during the reaction, the reaction can be carried out at a lower pressure. In this case, the reaction pressure is, for example, 10 to 150 atm, preferably 30 to 120 atm, and more preferably 50 to 100 atm.

In a method for producing an alcohol of one embodiment, the reaction time may be appropriately selected in consideration of the reaction temperature, the reaction pressure, the raw material conversion rate, etc. Specifically, it may be 0.1 to 10 hrs.

An alcohol may be produced by a batch method, a semi-batch method, or a continuous method.

In the method for producing an alcohol, the organic solvent containing the transition metal complex of Group 9 can be recovered by easily separating the product alcohol by a simple operation of distilling the reaction liquid after the reaction, distilling off the target alcohol, and obtaining the alcohol. The recovered organic solvent can be used as it is a solvent for the next reaction. That is, the second alcohol production reaction can be carried out by adding an olefin compound, carbon monoxide, and a hydrogen molecule to the reaction liquid after the alcohol distillation, and reacting the mixture. At this time, if necessary, an organic solvent containing a transition metal complex of Group 9 may be newly added.

The distillation method may be either a batch method or a continuous method as long as the product alcohol and a by-produced aldehyde are distilled off from the reaction liquid. The distillation method may be a simple distillation, a flash distillation, or a continuous distillation. The distillation may be carried out at normal pressure or under reduced pressure.

The distillation temperature can be appropriately selected depending on the target alcohol and the organic solvent used. The distillation temperature is, for example, less than 200°C. The range of distillation temperature is preferably 50°C or more and less than 200°C, more preferably 70°C to 180°C, and particularly preferably 80°C to 150°C. At a temperature of 200°C or more, the solvent may be distilled off, and the purity of the alcohol may be lowered, and at a temperature less than 50°C, the distillation of the product alcohol may be delayed.

When only the amine compound having two or more nitrogen atoms constituting a tertiary amine is used as the organic solvent used for the reaction, the catalyst liquid is composed only of the transition metal complex of Group 9 and the amine compound, and therefore, the composition of the catalyst liquid hardly changes before and after distillation of the alcohol. In addition, the activity of the catalyst is hardly reduced due to the stabilization by the amine compound. Therefore, even when the catalyst liquid is used a plurality of times, the alcohol can be stably produced. From this viewpoint, the content of the amine compound having two or more nitrogen atoms constituting a tertiary amine in the organic solvent is preferably 100 mol%.

At normal pressure, ethylene, a hydrogen molecule, and carbon monoxide are gases, the boiling point of 1-propanol is 97°C, the boiling point of propionaldehyde is 47°C, and the boiling point of N,N,N',N'-tetramethyl-1,3-propanediamine is about 145°C. Therefore, when the olefin compound is ethylene, the produced alcohol is 1-propanol, and the amine compound having two or more nitrogen atoms constituting a tertiary amine is N,N,N',N'-tetramethyl-1,3-propanediamine, for example, by distilling at about 120 to 130°C under normal pressure, only 1-propanol and propionaldehyde can be distilled off and obtained without distilling off the amine compound, and thus the catalyst liquid composed of the transition metal complex of Group 9 and the amine compound can be recovered. By adding ethylene, a hydrogen molecule, and carbon monoxide to the recovered catalyst liquid, 1-propanol can be produced again. 1-propanol and propionaldehyde can be easily separated by known methods, and propionaldehyde can be converted to 1-propanol by adding the propionaldehyde to the catalyst liquid.

### [Applications]

An alcohol obtained by the method for producing an alcohol is useful as a solvent and an intermediate for medical and agricultural chemicals.

### EXAMPLES

Hereinafter, the present invention will be described in more detail with reference to Examples and Comparative Examples, but the present invention is not limited to these Examples. Parts and percentages in the examples are based on mass unless otherwise specified.

Analysis, testing, and evaluation in Examples and Comparative Examples were carried out by the following methods.

### (1) Gas chromatography analysis of reaction liquid

Analysis of the reaction liquid after the reaction was carried out by gas chromatography.

### ·Gas chromatography conditions

Column oven: kept at 60°C for 12 minutes, then raised to 80°C at 10°C/min, and kept for 12 minutes, then raised to 150°C at 20°C/min, and kept for 10 minutes, then raised to 230°C at 30°C/min, and kept for 5 minutes, then raised to 235°C at 30°C/min, and kept for 10 minutes.
Carrier gas: helium
Vaporizing chamber temperature: 230°C
Detector temperature: 260°C

### ·Capillary column

J & W Ultra 2 (manufactured by Agilent Technologies Japan, Ltd, length 50 m, inner diameter 0.32 mm, film thickness 0.52 µm)

### · Detectors

### FID

### Example 1: Synthesis of 1-propanol from ethylene

5 mg (0.019 mmol) of acetylacetonatodicarbonylrhodium (Rh(acac)(CO)₂, manufactured by Aldrich) and 3.9 g (30 mmol) of N,N,N',N'-tetramethylpropane-1,3-diamine (TMPDA, manufactured by Aldrich) as the amine compound having two or more nitrogen atoms constituting a tertiary amine were placed in a 50 mL SUS autoclave, and the inside of the autoclave was purged with argon gas. Subsequently, the inside was purged with syngas (CO:H₂ = 1:1 molar ratio), then 0.91 g (32 mmol) of ethylene (C₂H₄), and a mixed gas of CO and H₂ were added at a total pressure of 90 atm so as to be C₂H₄:CO:H₂ = 1 mol:2.5 mol:2.5 mol, and the mixture was heated at 110°C for 3 hours with stirring. It was confirmed, from gas chromatography analysis results of the reaction liquid after the reaction, that only 1-propanol and propionaldehyde were produced. The total yield of 1-propanol and propionaldehyde was 88%, the selectivity of 1-propanol was 97%, and the selectivity of propionaldehyde was 3%. After the reaction, the reaction liquid was heated to 130°C and distilled at normal pressure to distill off 1-propanol and propionaldehyde, and a catalyst liquid (4.1 g) composed of Rh(acac)(CO)₂ and TMPDA was recovered. The results are shown in Table 1.

### Examples 2 to 4: Synthesis of 1-propanol from ethylene

The reaction was carried out in the same manner as in Example 1, except that the reaction temperature and pressure were changed as described in Table 1. The results are shown in Table 1.

### Example 5: Synthesis of 1-propanol from ethylene

The reaction was carried out in the same manner as in Example 1, except that the solvent was changed to a mixed solvent of N,N,N',N'-tetramethyl-1,3-propanediamine (30 mmol) and toluene (19 mmol) (amine compound 61 mol%). The total yield of 1-propanol and propionaldehyde was 89%, the selectivity of 1-propanol was 96%, and the selectivity of propionaldehyde was 4% from the results of gas chromatography analysis of the reaction liquid after the reaction. The results are shown in Table 1.

### Examples 6 to 7: Synthesis of 1-propanol from ethylene

The reaction was carried out in the same manner as in Example 5, except that n-decane (10 mmol) and n-butanol (22 mmol) were used respectively instead of toluene, and the contents of the amine compounds were changed to 75 mol% and 58 mol% respectively. The results are shown in Table 1.

### Example 8: Synthesis of 1-propanol from ethylene

The reaction was carried out in the same manner as in Example 5, except that the solvent was changed to a mixed solvent of N,N,N',N'-tetramethyl-1,3-propanediamine (12 mmol) and toluene (28 mmol) (amine compound 30 mol%). The results are shown in Table 1.

### Example 9: 1-propanol synthesis from ethylene

The reaction was carried out in the same manner as in Example 8, except that the amine compound was changed to N,N,N',N'-tetramethyl-1,4-butanediamine (TMBDA) and the content of the amine compound was changed to 31 mol%. The results are shown in Table 1.

### Example 10: 1-propanol synthesis from ethylene

The reaction was carried out in the same manner as in Example 8, except that the amine compound was changed to N,N,N',N'-tetramethyl-1,6-hexanediamine (TMHDA), and the content of the amine compound was changed to 34 mol%. The results are shown in Table 1.

### Example 11: Synthesis of 1-propanol from ethylene

The reaction was carried out in the same manner as in Example 8, except that the amine compound was changed to N,N,N',N",N"-pentamethyldiethylenetriamine (PMDETA), and the content of the amine compound was changed to 34 mol%. The results are shown in Table 1.

### Example 12

The reaction was carried out in the same manner as in Example 1, except that the catalyst liquid recovered in Example 1 was used, and TMPDA and Rh(acac)(CO) ₂ were not added. Almost the same reaction results as in Example 1 were obtained. The results are shown in Table 1.

### Example 13

A 50 mL SUS autoclave was charged with 3 mL of a toluene solution of Rh(acac)(CO)₂ (3.88 mmol/L) (0.012 mmol as Rh) and 2.3 g of TMPDA (18.0 mmol), and the inside of the autoclave was purged with argon gas. Subsequently, the inside was purged with syngas (CO:H₂ = 1:1 molar ratio), and then 5 atm of ethylene (0.27 g, 9.6 mmol) and a mixed gas of CO and H₂ were added at a total pressure of 30 atm so as to be C₂H₄:CO:H₂ = 5 mol:12.5 mol:12.5 mol. Thereafter, the mixture was heated with stirring for 3 hours, during which time a mixed gas of CO and H₂ (CO:H₂ = 1:1 molar ratio) was continuously fed so that the total pressure remained 30 atm. It was confirmed, from gas chromatography analysis results of the reaction liquid after the reaction, that only 1-propanol and propionaldehyde were produced. The total yield of 1-propanol and propionaldehyde was 100%, the selectivity of 1-propanol was 96%, and the selectivity of propionaldehyde was 4%.

### Comparative Example 1: Synthesis of 1-propanol from ethylene

The reaction was carried out in the same manner as in Example 8, except that the amine compound was changed to N,N,N',N'-tetramethylethylenediamine (TMEDA) and the content of the amine compound was changed to 28 mol%. The results are shown in Table 1.

### Comparative Example 2: Synthesis of 1-propanol from ethylene

The reaction was carried out in the same manner as in Example 8, except that the content of the amine compound was changed to 7 mol%. The results are shown in Table 1.

### Comparative Example 3

The reaction was carried out in the same manner as in Example 1, except that a solution of 607 mg (3.0 mmol) of tri-n-butylphosphine (P(nBu)₃) dissolved in toluene to make 5 mL was used as the solvent. The results are shown in Table 1. It was confirmed, from gas chromatography analysis results of the reaction liquid after the reaction, that only propionaldehyde was produced. The selectivity of 1-propanol was 0%.

### Comparative Example 4

The reaction was carried out in the same manner as in Example 1, except that 4.2 g (12 mmol) of triisooctylamine (TIOA) was used as the solvent. The results are shown in Table 1.

**Table 1**

| | | Solvent | | | Reaction conditions | | | | | | | | |
|---|---|---|---|---|---|---|---|---|---|---|---|---|---|
| | Olefin | Amine solvent | Other solvent | Content of amine solvent in solvent [mol%] | Amine solvent / Metal complex molar ratio | Olefin / Metal complex molar ratio | Temperature [°C] | Pressure [atm] | Time [h] | Total yield of aldehyde and alcohol [%] | 1-propanol selectivity [%] | Propionaldehyde selectivity [%] | ¹⁾ TOF¹⁾ [/h] |
| Ex. 1 | ethylene | TMPDA | - | 100 | 1579 | 1684 | 110 | 90 | 3 | 88 | 97 | 3 | 479 |
| Ex. 2 | ↑ | ↑ | - | 100 | ↑ | 1 | 70 | 1 | ↑ | 81 | 98 | 2 | 446 |
| 3 | ↑ | ↑ | - | 100 | ↑ | ↑ | 110 | 50 | ↑ | 96 | 87 | 13 | 469 |
| Ex. 4 | ↑ | ↑ | - | 100 | ↑ | ↑ | ↑ | 110 | ↑ | 83 | 74 | 26 | 345 |
| Ex. 5 | ↑ | t | toluene | 61 | ↑ | ↑ | ↑ | 90 | ↑ | 89 | 96 | 4 | 480 |
| Ex. 6 | ↑ | ↑ | decane | 75 | ↑ | ↑ | ↑ | ↑ | ↑ | 87 | 93 | 7 | 454 |
| Ex. 7 | ↑ | ↑ | n-butanol | 58 | ↑ | ↑ | ↑ | ↑ | ↑ | 90 | 95 | 5 | 480 |
| Ex. 8 | ↑ | ↑ | toluene | 30 | 632 | ↑ | ↑ | ↑ | ↑ | 88 | 97 | 3 | 479 |
| Ex. 9 | ↑ | TMBDA | ↑ | 31 | ↑ | ↑ | ↑ | ↑ | ↑ | 90 | 93 | 7 | 470 |
| Ex. 10 | ↑ | TMHDA | ↑ | 34 | ↑ | ↑ | ↑ | ↑ | ↑ | 88 | 96 | 4 | 474 |
| Ex. 11 | ↑ | PMDETA | ↑ | 34 | ↑ | ↑ | ↑ | ↑ | ↑ | 92 | 88 | 12 | 455 |
| Ex. 12 | ↑ | TMPDA | - | 100 | 1579 | ↑ | ↑ | ↑ | ↑ | 86 | 98 | 2 | 473 |
| Ex. 13 | ↑ | TMPDA | toluene | 49 | 1497 | 800 | 1 | 30 | ↑ | 100 | 96 | 4 | 256 |
| Comp. Ex. 1 | ↑ | TMEDA | toluene | 28 | **632** | 1684 | 110 | 90 | 3 | 98 | **49** | 51 | 270 |
| Comp. Ex. 2 | ↑ | TMPDA | ↑ | 7 | 150 | ↑ | ↑ | ↑ | ↑ | 84 | 26 | 74 | 123 |
| Comp. Ex. 3 | ↑ | P (nBu) 3 | 1 | 0 | 158²⁾ | ↑ | ↑ | ↑ | ↑ | 60 | 0 | 100 | 0 |
| Comp. Ex. 4 | ↑ | TIOA | ↑ | 100 | 632 | ↑ | ↑ | ↑ | ↑ | 69 | 9 | 91 | 35 |

| | | | | | | | | | | | | | |
|---|---|---|---|---|---|---|---|---|---|---|---|---|---|
| 1) TOF = Number of moles of produced alcohol [mol] / Number of moles of transition metal of Group 9 [mol] / Reaction time [h] 2) indicates phosphine ligand / metal complex molar ratio instead of amine solvent / metal complex molar ratio. | | | | | | | | | | | | | |

The TOF in the table represents the turnover frequency of the transition metal complex catalyst of Group 9 and is calculated by the following formula: TOF = Number of moles of produced alcohol [mol] / Number of moles of transition metal of Group 9 [mol] / Reaction time [h]. The higher the TOF, the higher the catalytic activity per unit time. The TOF in the Examples is 200 or more, and therefore the industrial alcohol production can be carried out with relatively small amounts of catalyst. Although propionaldehyde may be produced in the reaction of the Examples, 1-propanol and propionaldehyde can be easily separated by distillation. The obtained propionaldehyde is also useful as a food additive or a raw material for pharmaceuticals, and can be charged into a catalyst liquid to be used as a raw material for 1-propanol.

## Claims

1. A method for producing an alcohol comprising reacting an olefin compound, carbon monoxide, and a hydrogen molecule in an organic solvent containing 30 mol% or more of an amine compound having two or more nitrogen atoms constituting a tertiary amine, using a transition metal complex of Group 9 as a catalyst.

2. The method for producing an alcohol according to claim 1, wherein the amine compound having two or more nitrogen atoms constituting a tertiary amine is represented by formula (1) or formula (2): 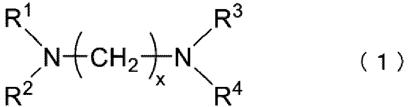 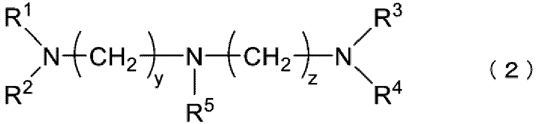 wherein, R¹ to R⁵ each independently represent an alkyl group having 1 to 10 carbon atoms, and x, y, and z each independently represent an integer of 2 to 10.

3. The method for producing an alcohol according to claim 2, wherein in formula (1) or (2), R¹ to R⁵ are all methyl groups or all ethyl groups, and x, y, and z are each independently an integer of 2 to 6.

4. The method for producing an alcohol according to claim 1, wherein the amine compound having two or more nitrogen atoms constituting a tertiary amine is at least one selected from the group consisting of N,N,N',N'-tetramethylethylenediamine, N,N,N',N'-tetramethyl-1,3-propanediamine, N,N,N',N'-tetramethyl-1,4-butanediamine, N,N,N',N'-tetramethyl-1,6-hexanediamine, and N,N,N',N",N"-pentamethyldiethylenetriamine.

5. The method for producing an alcohol according to claim 1, wherein the amine compound having two or more nitrogen atoms constituting a tertiary amine is N,N,N',N'-tetramethyl-1,3-propanediamine.

6. The method for producing an alcohol according to any one of claims 1 to 5, wherein the transition metal complex of Group 9 is a metal complex composed of acetylacetonatodicarbonyl and a transition metal of Group 9.

7. The method for producing an alcohol according to claim 6, wherein the transition metal of Group 9 is rhodium.

8. The method for producing an alcohol according to any one of claims 1 to 5, wherein the organic solvent containing 30 mol% or more of the amine compound having two or more nitrogen atoms constituting a tertiary amine is an organic solvent comprising the amine compound having two or more nitrogen atoms constituting a tertiary amine, and at least one selected from the group consisting of toluene, xylene, and n-butanol.

9. The method for producing an alcohol according to any one of claims 1 to 5, wherein the molar ratio of the amine compound having two or more nitrogen atoms constituting a tertiary amine to the transition metal of Group 9 is 500 to 10,000.

10. The method for producing an alcohol according to any one of claims 1 to 5, wherein the olefin compound is a monoolefin having 2 to 4 carbon atoms.

11. The method for producing an alcohol according to any one of claims 1 to 5, wherein the olefin compound is ethylene and the product is 1-propanol.

12. The method for producing an alcohol according to any one of claims 1 to 5, comprising separating a produced alcohol after the reaction, recovering the organic solvent containing the transition metal complex of Group 9, adding an olefin compound, carbon monoxide, and a hydrogen molecule to the recovered organic solvent, and reacting the mixture.

13. The method for producing an alcohol according to claim 12, comprising distilling off the produced alcohol by distillation at a temperature of less than 200°C, and recovering the organic solvent containing a transition metal complex of Group 9.
